# EUROPEAN PATENT SPECIFICATION

(11) **EP 3 905 991 B1**
(45) Date of publication and mention of the grant of the patent: **18.11.2026**
(21) Application number: 19829686.5
(22) Date of filing: 17.12.2019
(51) Int. Cl.: A61F 2/34

(54) **ACETABULAR ORTHOPAEDIC PROSTHESIS**
ORTHOPÄDISCHE HÜFTGELENKPROTHESE
PROTHÈSE ORTHOPÉDIQUE ACÉTABULAIRE

(30) Priority: 31.12.2018 US 201816236801
(43) Date of publication of application: 10.11.2021
(73) Proprietor: DePuy Ireland Unlimited Company, Ringaskiddy, County Cork (IE)
(72) Inventor: LEWIS, Paul P., Raynham, Massachusetts 02767-0350 (US); CAYWOOD, James A., Raynham, Massachusetts 02767-0350 (US); SCHMALZRIED, Thomas P., Los Angeles, California 90057 (US); BRANDEWIE, Alena M., Raynham, Massachusetts 02767-0350 (US)
(74) Representative: Murgitroyd & Company
(86) International application number: PCT/IB2019/060875
(87) International publication number: WO 2020/141383

(56) References cited:
- US-A- 3 829 904
- US-A- 5 133 763
- US-A- 5 458 649
- US-A- 5 549 691

## Description

### TECHNICAL FIELD

The present disclosure relates generally to orthopaedic prosthetic components and, more particularly, to acetabular prosthetic components.

### BACKGROUND

Joint arthroplasty is a well-known surgical procedure by which a diseased and/or damaged natural joint is replaced by a prosthetic joint. For example, in a hip arthroplasty surgical procedure, a patient's natural hip ball and socket joint is partially or totally replaced by a prosthetic hip joint. A typical prosthetic hip joint includes an acetabular prosthetic component and a femoral head prosthetic component. An acetabular prosthetic component generally includes an outer shell configured to engage the acetabulum of the patient and an inner bearing or insert coupled to the shell and configured to engage the femoral head. The femoral head prosthetic component and inner insert of the acetabular component form a ball and socket joint that approximates the natural hip joint.

US3829904 provides an orthopaedic prosthesis according to the preamble of the claimed invention and US5458649 and US5549691 disclose similar structures.

### SUMMARY

An orthopaedic prosthetic component according to the invention is defined in claim 1.

In some embodiments, the outer rim may include an inferior rim section and a superior rim section that extends at a non-orthogonal angle relative to the inferior rim section. In some embodiments, the non-orthogonal angle may be equal to about 15 degrees.

In some embodiments, the acetabular prosthetic component may include an outer wall that is positioned opposite the inner wall. The outer wall may include a lateral edge that is positioned lateral of the imaginary plane.

In some embodiments, the acetabular prosthetic component may include a component axis that extends orthogonal to the inferior rim section, and the semi-spherical surface may include a central axis that extends at a non-orthogonal angle relative to the component axis. Additionally, in some embodiments, the non-orthogonal angle may be equal to about 15 degrees.

In some embodiments, the cylindrical surface may include a second central axis that is coincident with the central axis of the semi-spherical surface.

Additionally, in some embodiments, the orthopaedic prosthetic component may also comprises a prosthetic shell including an outer surface shaped to be positioned in a surgically-prepared acetabulum of a patient's pelvis and an inner cavity that receives the acetabular prosthetic component.

In some embodiments, the prosthetic shell may include a second central axis that is coincident with the component axis of the acetabular prosthetic component.

### BRIEF DESCRIPTION OF THE DRAWINGS

The detailed description particularly refers to the following figures, in which:
FIG. 1 is a perspective view of an orthopaedic prosthesis for use in a hip arthroplasty;
FIG. 2 is an exploded perspective view of an acetabular prosthesis and a femoral head component of the orthopaedic prosthesis;
FIG. 3 is a side elevation view of an insert component of the acetabular prosthesis of FIG. 2;
FIG. 4 is a cross-sectional elevation view of the insert component taken along the line 4-4 in FIG. 2; and
FIG. 5 is a cross-sectional elevation view similar to FIG. 4 with the insert component coupled to a prosthetic shell component of the acetabular prosthesis of FIG. 2.

### DETAILED DESCRIPTION OF THE DRAWINGS

While the concepts of the present disclosure are susceptible to various modifications and alternative forms, specific exemplary embodiments thereof have been shown by way of example in the drawings and will herein be described in detail. It should be understood, however, that there is no intent to limit the concepts of the present disclosure to the particular forms disclosed, but on the contrary, the intention is to cover all modifications and alternatives falling within the scope of the invention as defined by the appended claims.

Terms representing anatomical references, such as anterior, posterior, medial, lateral, superior, inferior, etcetera, may be used throughout the specification in reference to the orthopaedic implants or prostheses and surgical instruments described herein as well as in reference to the patient's natural anatomy. Such terms have well-understood meanings in both the study of anatomy and the field of orthopaedics. Use of such anatomical reference terms in the written description and claims is intended to be consistent with their well-understood meanings unless noted otherwise.

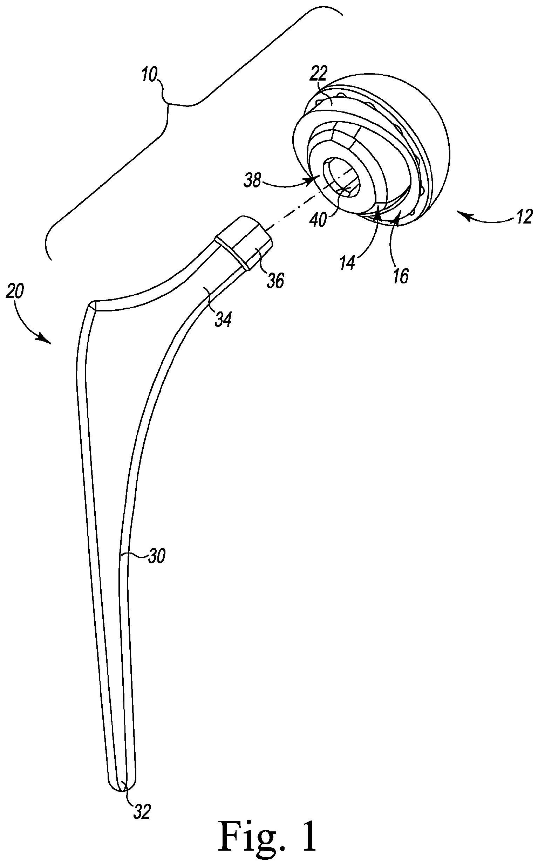

Referring now to FIG. 1, a hip orthopaedic prosthesis assembly 10 includes a plurality of components, including an acetabular prosthesis 12 that is sized to be implanted in a surgically-prepared acetabulum of a patient's pelvis. The assembly 10 also includes a femoral head component 14 that is received in a cavity 16 defined in the acetabular prosthesis 12. The prosthesis assembly 10 also includes a femoral stem component 20 that is configured to be secured to the femoral head component 14 to form a femoral prosthetic component. As described in greater detail below, the acetabular prosthesis 12 includes an insert component 22 that is shaped to support and stabilize the femoral head 14 over the range of motion of the prosthesis assembly 10.

In the illustrative example, the femoral stem component 20 includes an elongated body 30 that extends from a distal tip 32. The stem component 20 is sized and shaped to be implanted in a surgically-prepared proximal end of a patient's femur. The stem component 20 also includes a neck 34 that extends superiorly and immediately from the elongated body 30 to a tapered trunnion 36. The tapered trunnion 36 is sized to be positioned in a distal bore 38 defined in the femoral head component 14. The bore 38 is defined by a tapered inner surface 40 that is configured to engage the tapered trunnion 36 to secure the head component 14 to the stem component 20. The femoral head component 14 and the femoral stem component 20 are separately formed from implant-grade metallic materials such as, for example, titanium.

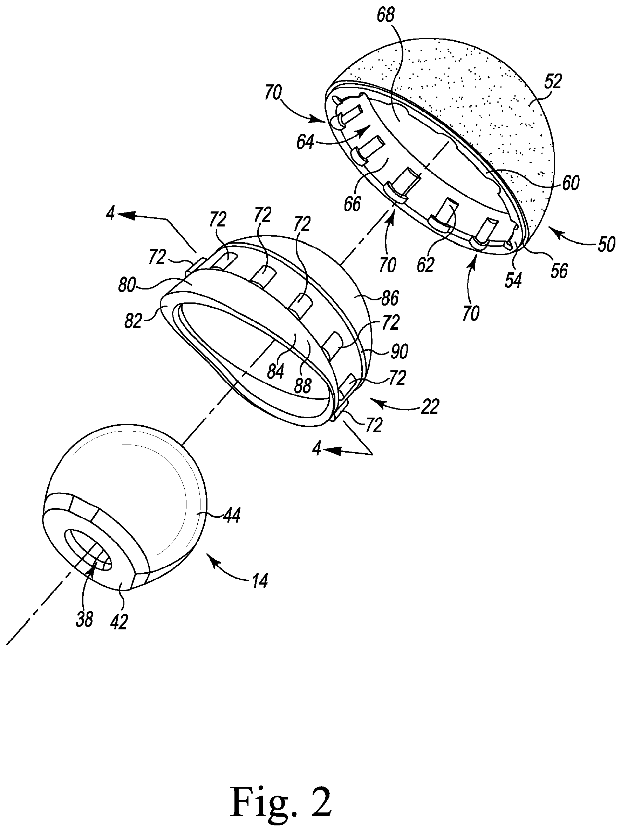

Referring now to FIG. 2, the bore 38 of the femoral head component 14 is defined in a distal surface 42. The head component 14 also includes a convex curved surface 44 that is connected to, and extends from, the distal surface 42. In the illustrative embodiment, the convex curved surface 44 is a semi-spherical surface that is shaped to be received in the cavity 16 of the acetabular prosthesis 12.

As described above, the acetabular prosthesis 12 includes an insert component 22, which is configured to be coupled to a shell component 50 of the acetabular prosthesis 12. The insert component 22 is illustratively formed from a polymeric material such as, for example, polyethylene. The shell component 50, is separately formed from implant-grade metallic materials such as, for example, titanium. The shell component 50 also includes a Porocoat^{®} outer coating 52 that permits bone to affix biologically to the shell component 50 after implantation. It should be appreciated that in other embodiments the Porocoat^{®} outer coating may be omitted.

The shell component 50 has a distal rim 54 and an outer wall 56 that extends from the distal rim 54. The outer wall 56 includes a convex curved outer surface and an annular outer surface 60 that extends from the distal rim 54 to the curved outer surface. In the illustrative example, the convex curved outer surface is semi-spherical and shaped to match the shape of a patient's surgical prepared acetabulum. The Porocoat^{®} outer coating 52 covers the outer surface and follows its geometric shape.

An inner wall 62 extends inwardly from the distal rim 54 to define a cavity 64 in the shell component 50 that is sized to receive the insert component 22. The inner wall 62 includes an annular inner surface 66 that is positioned opposite the annular outer surface 60 and a concave curved inner surface 68 that is connected to the annular inner surface 66. A plurality of slots 70, which extend inwardly form the distal rim 54, are defined in the inner wall 62. The slots 70 are spaced apart around the circumference of the distal rim 54 and are shaped to receive corresponding keys 72 of the insert component 22, as described in greater detail below.

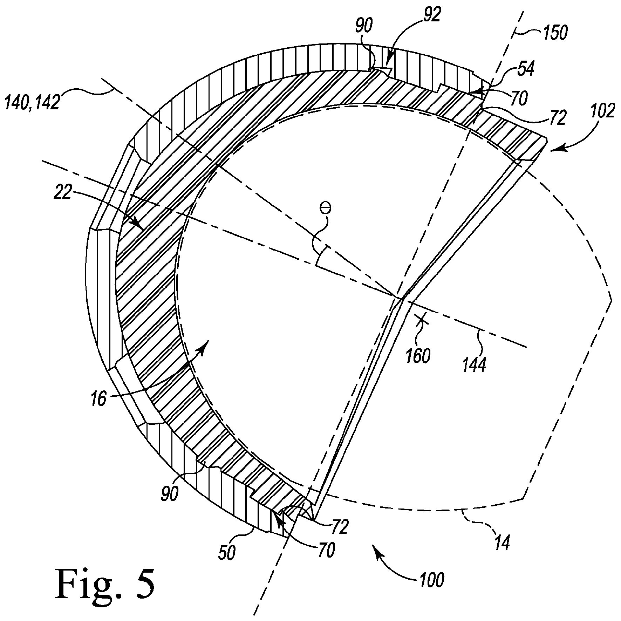

The insert component 22 includes a body 80 that is shaped to be received in the cavity 64 of the shell component 50. The body 80 includes an outer rim 82 and an outer wall 84 that extends from the outer rim 82. The outer wall 84 includes a convex curved outer surface 86 and an annular outer surface 88 that extends from the outer rim 82 to the curved outer surface 86. The keys 72 extend outwardly from, and are positioned around the outer circumference of, the annular outer surface 88. The insert component 22 also includes an annular rib 90 that is formed on the convex curved outer surface 86 and is sized to be received in a corresponding annular slot 92 (see FIG. 5) formed in the concave curved inner surface 68 of the shell component 50. The rib 90 engages the shell component 50 to secure the insert component 22 to the shell component 50. It should be appreciated that in other embodiments the rib may be included on the shell component and the slot on the insert component.

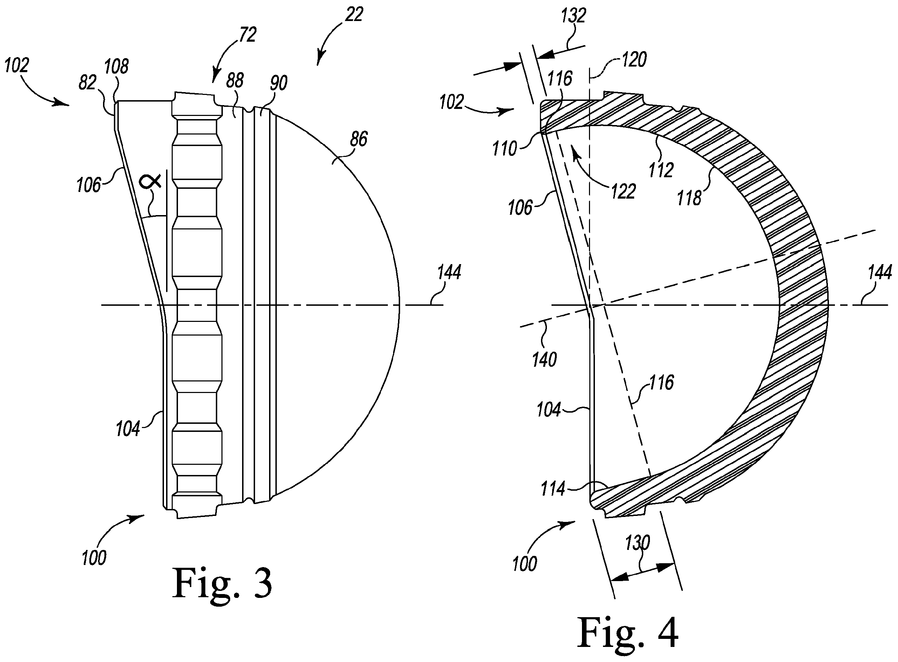

Referring now to FIG. 3, the insert component 22 has a side 100 that is positioned inferiorly when the acetabular prosthesis 12 is placed at in the proper orientation in a patient's acetabulum and another side 102 that is positioned superiorly. The outer rim 82 includes a rim section 104 that is positioned on the inferior side 100 of the insert component 22 and another rim section 106 that is connected to the rim section 104 and extends to the superior side 102 of the insert component 22. As shown in FIG. 3, the superior rim section 106 is connected to a lateral edge 108 of the outer wall 84 of the insert component 22.

When the insert component 22 is coupled to the shell component 50, the inferior rim section 104 extends parallel to the distal rim 54 of the shell component 50. The superior rim section 106 is sloped relative to the inferior rim section 104 such that an angle α is defined between the sections 104, 106. The angle α is a non-orthogonal angle that is greater than 90 degrees. In the illustrative embodiment, the angle α is equal to about 15 degrees. It should be appreciated that the term "about" as used herein denotes a range of ± 2% or less of the base number. As such, "about 15 degrees" may be understood as a range including ± 2% of 15 degrees. It should also be appreciated that in other embodiments the angle α may be in a range of about 15 to about 30 degrees.

As described above, the acetabular prosthesis 12 includes a cavity 16 that is sized to receive the femoral head component 14. The cavity 16 has an opening 110 that is defined by the outer rim 82 of the insert component 22. As shown in FIG. 4, the insert component 22 includes an inner wall 112 that extends inwardly from the outer rim 82 to define the cavity 16. The inner wall 112 includes a curved surface 114 that extends from the outer rim 82 to an inner end 116 (partially indicated by the broken line in FIG. 4). The inner wall 112 also includes a semi-spherical surface 118 that is connected to the inner end 116 of the curved surface 114. The semi-spherical surface 118 is a concave curved surface that is shaped to match the semi-spherical convex surface 44 of the femoral head component 14. In use, the femoral head component 14 articulates on the semi-spherical surface 118 of the insert component 22.

As shown in FIG. 4, the inferior rim section 104 is positioned in an imaginary plane 120 that extends through the inner wall 112. The semi-spherical surface 118 extends through the imaginary plane 120 on the superior side 102 of the insert component 22 to define a cupped superior region 122 that resists dislocation of the femoral head component 14 from the cavity 16 and thereby contributes to the retention of the femoral head component 14 in the cavity 16. The semi-spherical surface 118 connects to the inner end 116 on the superior side 102 along a line that is lateral of the imaginary plane 120.

According to the invention, the curved surface 114 is a cylindrical surface to define a distal bore. When viewed in cross-section as shown in FIG. 4, the cylindrical surface 114 extends a distance 130 on the inferior side 100 of the insert component 22 and extends a distance 132 on the superior side 102 of the insert component 22. The distance 132 is less than the distance 130 such that the superior depth of the distal bore is less than the inferior depth of the bore. The cylindrical surface 114 has a central axis 140 that extends coincidently with a central axis 142 of the semi-spherical surface 118.

Referring now to FIG. 5, the shell component 50 has a central axis 144. When the insert component 22 is coupled to the shell component 50, the central axis 144 extends at an angle θ relative to the axes 140, 142. In the illustrative embodiment, the angle θ is equal to about 15 degrees. It should be appreciated that in other embodiments the angle θ may be in a range of about 15 to about 30 degrees.

The distal rim 54 of the shell component 50 is positioned in an imaginary plane 150 that extends parallel to the imaginary plane 120 in which the inferior section 104 of the insert component 22 is positioned. As shown in FIG. 5, the superior side 102 of the insert component 22 extends through the imaginary plane 150 such that the lateral edge 108 of the outer wall 84 on that side of the insert component 22 is positioned lateral of the imaginary plane 150.

The femoral head component 14 has a geometric center 160 in the illustrative embodiment. As shown in FIG. 5, the cavity 16 is sized such that the geometric center 160 is positioned lateral of the imaginary plane 150 when the femoral head component 14 is properly seated in the cavity 16. In that way, the position of the femoral head component 14 is lateralized to permit a greater range of motion for the femoral components 14, 20 relative to the acetabular prosthesis 12.

In use, a surgeon may surgically prepare the patient's natural acetabulum to receive an acetabular prosthesis 12. To do so, the surgeon may utilize a surgical reamer to prepare the patient's bone to receive the prosthesis 12. The surgeon may also utilize one or more surgical trial components to determine which prosthesis is appropriate for the patient. For example, it should be appreciated that the surgeon may select an appropriately-sized shell component 50 and insert component 22 from a number of shell components and insert components of different sizes. The surgeon may insert the shell component 50 into the patient's surgically-prepared acetabulum, advancing the shell component 50 into engagement with the concave bone surface defining the acetabulum. When the shell component 50 is oriented at a desired anteversion and inclination, the surgeon may secure the shell component 50 to the patient's bone.

To assemble the prosthesis 12, the surgeon may align the selected insert component 22 with the cavity 64 defined in the shell component 50 and advance the insert component 22 along the axis 144 to position the convex curved outer surface 86 of the insert component 22 in the cavity 64. The surgeon may rotate the insert component 22 about the axis 144 to align the keys 72 with the slots 70 defined in the shell component 50 and place the rim section 106 of the insert component 22 superior to the rim section 104. Once properly aligned, the surgeon may advance the keys 72 into the slots 70 and engage the rib 90 of the insert component 22 with the slot 92 of the shell component 50 to couple the insert component 22 to the shell component 50.

While the disclosure has been illustrated and described in detail in the drawings and foregoing description, such an illustration and description is to be considered as exemplary and not restrictive in character, it being understood that only illustrative embodiments have been shown and described and that all changes and modifications that come within the scope of the invention are desired to be protected.

There are a plurality of advantages of the present disclosure arising from the various features of the method, apparatus, and system described herein. It will be noted that alternative embodiments of the method, apparatus, and system of the present disclosure may not include all of the features described yet still benefit from at least some of the advantages of such features. Those of ordinary skill in the art may readily devise their own implementations of the method, apparatus, and system that incorporate one or more of the features of the present invention and fall within the scope of the invention, which is defined by the appended claims.

## Claims

1. An orthopaedic prosthetic component, comprising:
an acetabular prosthetic component [22] including an outer rim [82], an inner wall [112] extending inwardly from the outer rim [82], and a cavity [16] defined by the inner wall [112], the cavity [16] being sized to receive a femoral head [44] of a femoral prosthetic component [14] and comprising a semi-spherical surface [118], wherein the outer rim [82] includes an inferior rim section [104] that is positioned in an imaginary plane [120] such that a portion of the semi-spherical surface [118] on a superior side [102] extends through the imaginary plane [120], **characterised in that**
the inner wall [112] includes a cylindrical surface [114] extending inwardly from the outer rim [82] to an inner end [116], wherein the semi-spherical surface [118] is connected to the inner end [116] of the cylindrical surface [114],
wherein, when the acetabular prosthetic component [22] is viewed in cross-section, the cylindrical surface [114] extends a first distance [130] from the outer rim [82] to the inner end [116] on an inferior side [100] of the acetabular prosthetic component [22] and the cylindrical surface [114] extends a second distance [132] from the outer rim [82] to the inner end [116] on the superior side [102] of the acetabular prosthetic component [22], the second distance [132] being less than the first distance [130].

2. The orthopaedic prosthetic component of claim 1, wherein the outer rim [82] includes the inferior rim section [104] and a superior rim section [106] that extends at a non-orthogonal angle relative to the inferior rim section [104].

3. The orthopaedic prosthetic component of claim 1, wherein the non-orthogonal angle is equal to about 15 degrees.

4. The orthopaedic prosthetic component of claim 1, wherein the acetabular prosthetic component [22] includes an outer wall [84] that is positioned opposite the inner wall [112], the outer wall [84] including a lateral edge [108] that is positioned lateral of the imaginary plane [120].

5. The orthopaedic prosthetic component of claim 2, wherein:
the acetabular prosthetic component [22] includes a component axis [144] that extends orthogonal to the inferior rim section [104], and
the semi-spherical surface [118] includes a central axis [140] that extends at a non-orthogonal angle relative to the component axis [144].

6. The orthopaedic prosthetic component of claim 5, wherein the non-orthogonal angle is equal to about 15 degrees.

7. The orthopaedic prosthetic component of claim 5, wherein the cylindrical surface [114] includes a second central axis that is coincident with the central axis [140] of the semi-spherical surface [118].

8. The orthopaedic prosthetic component of claim 5, further comprising a prosthetic shell [50] including an outer surface shaped to be positioned in a surgically-prepared acetabulum of a patient's pelvis and an inner cavity [64] that receives the acetabular prosthetic component [22].

9. The orthopaedic prosthetic component of claim 8, wherein the prosthetic shell [50] includes a second central axis that is coincident with the component axis [144] of the acetabular prosthetic component [22].

## Patentansprüche

1. Eine orthopädische Prothesenkomponente, die Folgendes beinhaltet:
eine Hüftpfannenprothesenkomponente [22], die einen äußeren Rand [82], eine Innenwand [112], die sich von dem äußeren Rand [82] nach innen erstreckt, und einen Hohlraum [16], der durch die Innenwand [112] definiert ist, umfasst, wobei der Hohlraum [16] bemessen ist, um einen Femurkopf [44] einer Femurprothesenkomponente [14] aufzunehmen, und eine halbkugelförmige Oberfläche [118] beinhaltet, wobei der äußere Rand [82] einen unteren Randabschnitt [104] umfasst, der in einer gedachten Ebene [120] positioniert ist, sodass sich ein Abschnitt der halbkugelförmigen Oberfläche [118] auf einer oberen Seite [102] durch die gedachte Ebene [120] erstreckt, **dadurch gekennzeichnet, dass** die Innenwand [112] eine zylindrische Oberfläche [114] umfasst, die sich von dem äußeren Rand [82] nach innen zu einem inneren Ende [116] erstreckt, wobei die halbkugelförmige Oberfläche [118] mit dem inneren Ende [116] der zylindrischen Oberfläche [114] verbunden ist,
wobei, wenn die Hüftpfannenprothesenkomponente [22] im Querschnitt betrachtet wird, sich die zylindrische Oberfläche [114] um einen ersten Abstand [130] von dem äußeren Rand [82] zu dem inneren Ende [116] auf einer unteren Seite [100] der Hüftpfannenprothesenkomponente [22] erstreckt und sich die zylindrische Oberfläche [114] um einen zweiten Abstand [132] von dem äußeren Rand [82] zu dem inneren Ende [116] auf der oberen Seite [102] der Hüftpfannenprothesenkomponente [22] erstreckt, wobei der zweite Abstand [132] kleiner als der erste Abstand [130] ist.

2. Orthopädische Prothesenkomponente gemäß Anspruch 1, wobei der äußere Rand [82] den unteren Randabschnitt [104] und einen oberen Randabschnitt [106] umfasst, der sich in einem nicht orthogonalen Winkel relativ zu dem unteren Randabschnitt [104] erstreckt.

3. Orthopädische Prothesenkomponente gemäß Anspruch 1, wobei der nicht orthogonale Winkel gleich etwa 15 Grad ist.

4. Orthopädische Prothesenkomponente gemäß Anspruch 1, wobei die Hüftpfannenprothesenkomponente [22] eine Außenwand [84] umfasst, die gegenüber der Innenwand [112] positioniert ist, wobei die Außenwand [84] eine laterale Kante [108] umfasst, die lateral von der gedachten Ebene [120] positioniert ist.

5. Orthopädische Prothesenkomponente gemäß Anspruch 2, wobei:
die Hüftpfannenprothesenkomponente [22] eine Komponentenachse [144] umfasst, die sich orthogonal zu dem unteren Randabschnitt [104] erstreckt, und
die halbkugelförmige Oberfläche [118] eine zentrale Achse [140] umfasst, die sich in einem nicht orthogonalen Winkel relativ zu der Komponentenachse [144] erstreckt.

6. Orthopädische Prothesenkomponente gemäß Anspruch 5, wobei der nicht orthogonale Winkel gleich etwa 15 Grad ist.

7. Orthopädische Prothesenkomponente gemäß Anspruch 5, wobei die zylindrische Oberfläche [114] eine zweite zentrale Achse umfasst, die mit der zentralen Achse [140] der halbkugelförmigen Oberfläche [118] zusammenfällt.

8. Orthopädische Prothesenkomponente gemäß Anspruch 5, die ferner eine Prothesenschale [50] beinhaltet, die eine äußere Oberfläche, die geformt ist, um in einer chirurgisch vorbereiteten Hüftpfanne des Beckens eines Patienten positioniert zu werden, und einen inneren Hohlraum [64], der die Hüftpfannenprothesenkomponente [22] aufnimmt, umfasst.

9. Orthopädische Prothesenkomponente gemäß Anspruch 8, wobei die Prothesenschale [50] eine zweite zentrale Achse umfasst, die mit der Komponentenachse [144] der Hüftpfannenprothesenkomponente [22] zusammenfällt.

## Revendications

1. Un composant prothétique orthopédique, comprenant :
un composant prothétique acétabulaire [22] incluant un rebord extérieur [82], une paroi intérieure [112] s'étendant vers l'intérieur depuis le rebord extérieur [82], et une cavité [16] définie par la paroi intérieure [112], la cavité [16] étant dimensionnée pour recevoir une tête fémorale [44] d'un composant prothétique fémoral [14] et comprenant une surface semi-sphérique [118], où le rebord extérieur [82] inclut une section de rebord inférieure [104] qui est positionnée dans un plan imaginaire [120] de telle sorte qu'une portion de la surface semi-sphérique [118] sur un côté supérieur [102] s'étend à travers le plan imaginaire [120], **caractérisé en ce que**
la paroi intérieure [112] inclut une surface cylindrique [114] s'étendant vers l'intérieur depuis le rebord extérieur [82] jusqu'à une extrémité intérieure [116], où la surface semi-sphérique [118] est reliée à l'extrémité intérieure [116] de la surface cylindrique [114],
où, lorsque le composant prothétique acétabulaire [22] est vu en coupe transversale, la surface cylindrique [114] s'étend sur une première distance [130] depuis le rebord extérieur [82] jusqu'à l'extrémité intérieure [116] sur un côté inférieur [100] du composant prothétique acétabulaire [22] et la surface cylindrique [114] s'étend sur une deuxième distance [132] depuis le rebord extérieur [82] jusqu'à l'extrémité intérieure [116] sur le côté supérieur [102] du composant prothétique acétabulaire [22], la deuxième distance [132] étant plus petite que la première distance [130].

2. Le composant prothétique orthopédique de la revendication 1, où le rebord extérieur [82] inclut la section de rebord inférieure [104] et une section de rebord supérieure [106] qui s'étend selon un angle non orthogonal relativement à la section de rebord inférieure [104].

3. Le composant prothétique orthopédique de la revendication 1, où l'angle non orthogonal est égal à environ 15 degrés.

4. Le composant prothétique orthopédique de la revendication 1, où le composant prothétique acétabulaire [22] inclut une paroi extérieure [84] qui est positionnée à l'opposé de la paroi intérieure [112], la paroi extérieure [84] incluant un bord latéral [108] qui est positionné latéralement par rapport au plan imaginaire [120].

5. Le composant prothétique orthopédique de la revendication 2, où :
le composant prothétique acétabulaire [22] inclut un axe de composant [144] qui s'étend orthogonalement à la section de rebord inférieure [104], et
la surface semi-sphérique [118] inclut un axe central [140] qui s'étend selon un angle non orthogonal relativement à l'axe de composant [144].

6. Le composant prothétique orthopédique de la revendication 5, où l'angle non orthogonal est égal à environ 15 degrés.

7. Le composant prothétique orthopédique de la revendication 5, où la surface cylindrique [114] inclut un deuxième axe central qui coïncide avec l'axe central [140] de la surface semi-sphérique [118].

8. Le composant prothétique orthopédique de la revendication 5, comprenant en outre une coque prothétique [50] incluant une surface extérieure formée pour être positionnée dans un acétabulum préparé chirurgicalement du bassin d'un patient et une cavité intérieure [64] qui reçoit le composant prothétique acétabulaire [22].

9. Le composant prothétique orthopédique de la revendication 8, où la coque prothétique [50] inclut un deuxième axe central qui coïncide avec l'axe de composant [144] du composant prothétique acétabulaire [22].
